# EUROPEAN PATENT APPLICATION

(11) **EP 4 011 315 A1**
(43) Date of publication of application: **15.06.2022**
(21) Application number: 21212851.6
(22) Date of filing: 07.12.2021
(51) Int. Cl.: A61B 34/20, A61B 34/00, G06N 20/00, A61J 15/00, A61B 90/00, A61B 34/10

(54) **GUIDANCE SYSTEM WITH CLAVICULAE POSITION SENSORS**

(30) Priority: 08.12.2020 US 202017115078
(71) Applicant: Envizion Medical Ltd., 6971060 Tel Aviv (IL)
(72) Inventor: BESSER, Doron, 6935927 Tel Aviv (IL); COHEN, Ran, 4972626 Petah-Tikva (IL); BEN EZRA, Guy, 3707992 Karkur (IL); COHEN, Amit, 4972626 Petah Tikva (IL); HILLMAN, Ayelet, 3824261 Hadera (IL); HOFSHI, Anat, 6274403 Tel Aviv (IL)
(74) Representative: AWA Denmark A/S

(57) **Abstract**

A tube positioning guidance system including an electromagnetic field generator configured to generate an electromagnetic field covering a treatment area; at least two reference sensors configured for positioning on the subject's upper torso and to sense the electromagnetic field; a registration sensor configured to sense the electromagnetic field and, wherein the registration sensor is utilized to mark a anatomic locations on the subject's torso; and a processing circuitry configured to determine the position, direction and/or insertion path of an enteral tube relative to the subject's suprasternal notch and xiphoid process based on signals obtained from the sensors and changes in the strength of the electromagnetic field sensed by the enteral tube.

## Description

### FIELD OF INVENTION

Embodiments of the disclosure relate to device, system and method for guiding tube positioning, specifically device, system and method for guiding tube positioning based on sensing of changes in an electromagnetic field using sensors positions on a subject's upper torso.

### BACKGROUND

Enteral feeding is often used as nutritional support in patients unable to be fed otherwise. Although many benefits are associated with early initiation of enteral feeding, misplacement of feeding tubes is relatively common and can result in patient discomfort and complications. Confirming the position of the tube only after it is already inserted delays the feeding and the initiating of hydration or medication. Bedside electromagnetic (EM) systems for guided placement of naso/enteral feeding tubes are available and are utilized by medical staff during the procedure to avoid misplacement of feeding tubes.

There is therefore a need for reliable real-time electromagnetic-based tracking systems that provide enhanced accuracy for critical tool positioning during medical procedures.

### SUMMARY

One of the problems often associated with an insertion of a feeding tube using an electromagnetic positioning guidance system, is that reliability is difficult to obtain in the patient environment, which is typically dynamic. This problem is further enhanced when the patient is moving, sweating and/or when specific instructions need be meticulously followed caregivers.

According to some embodiments, there is provided a tube positioning device comprising: a processing circuitry configured to track insertion of an insertion tube (e.g. a feeding tube) based on changes in an electromagnetic field generated over the patient's body, the changes caused by an electromagnetic sensor positioned on the tube. The tube positioning device includes a pair of reference sensors for positioning on the subject's torso, e.g. at or near the patient's claviculae.

Advantageously, the marking of the side of the torso using the registration sensor, obviates the need for a reference sensor positioned on the side of the subject's torso, as in previously disclosed system which tend to move as a result of patient movement, sweating etc.

In addition, the hereindisclosed tube positioning device does not require that the registration sensor of the device be maintained in a strictly upright position (90 degrees to the subject's torso) when marking the xiphoid process, and as such is much less prone to inaccuracies caused during marking of anatomical locations.

Accordingly, the hereindisclosed device may advantageously provide reliable and efficient guiding of the insertion in patient while requiring minimal training of users and being less prone to inaccuracies.

According to some embodiments, there is provided an enteral tube positioning guidance system comprising: an electromagnetic field generator configured to generate an electromagnetic field covering a treatment area; wherein said electromagnetic field generator is external to the patient; at least two wired or wirelessly connected reference sensors configured for positioning at a location on the subject's upper torso, wherein the at least two reference sensors are configured to sense the electromagnetic field generated by the field generator; a registration sensor configured to sense the electromagnetic field and to transmit information indicative of its location and/or orientation, wherein the registration sensor is utilized to mark a location of at least a xiphoid process and a side of the subject's torso; and a processing circuitry. According to some embodiments, the marking of the location of at least a xiphoid process and/or a side of the subject's torso may be automatic.

According to some embodiments, the processing circuitry configured to calculate a width of the subject's torso based on the location of the xiphoid process and the side of the subject's torso marked by the registration sensor; determine a plane defined by the at least two reference sensors, and the xiphoid process marked by the registration sensor; calculate a vector N normal to the plane; generating a diagram of the subject's upper body indicating the location of the subject's xiphoid process and suprasternal notch, wherein the diagram takes into consideration the calculated width of the subject's torso and the vector N.

According to some embodiments, calculating the width comprises calculating the distance between a point on the side of the subject's torso (marked by the registration sensor and/or automatically by the processor using one or more algorithms) and the line between the xiphoid process (marked by the registration sensor) and the suprasternal notch (either marked by the registration sensor or determined as further elaborated hereinbelow)

According to some embodiments, the processing circuitry is configured to determine in real-time, by changes in the strength of the electromagnetic field sensed by the electromagnetic sensor, a location, direction and/or path of a tip of the enteral tube relative to one or both of the pair of reference sensors, and projecting the location, direction and/or path over the diagram, using vector N to determine the projection direction.

According to some embodiments, the enteral tube may be a gastro-enteral tube. According to some embodiments, the enteral tube may be a naso-enteral tube. According to some embodiments, the enteral tube may be an oro-enteral tube. According to some embodiments, the enteral tube may be a feeding tube.

According to some embodiments, the location on the subject's upper torso where the reference sensors are positioned is at or near the subject's claviculae.

According to some embodiments, the diagram shows a frontal upper view of the subject's torso, a side view of the subject's torso and/or an axial view of the subject's torso as well as the insertion path of the tube in each view, as calculated by tracking the location of the tip sensor.

According to some embodiments, the system further includes a monitor configured to display the diagram. According to some embodiments, the diagram may be displayed on a mobile device. According to some embodiments, the diagram may be displayed on a monitor of another medical device, such as but not limited to a heart rate monitor or a monitor of a capnograph.

According to some embodiments, the registration sensor is incorporated into a tip of a stylus configured to be manually operated.

According to some embodiments, the registration sensor is further configured to mark the suprasternal notch. Alternatively, the processing circuitry may be configured to calculate the location of the suprasternal notch, based on the signals obtained from the at least two reference sensors.

According to some embodiments, there is provided a method for guiding insertion of a enteral tube into a subject, the method comprising: applying an electromagnetic field covering at least part of a subject's torso utilizing an external electromagnetic field generator; positioning at least two wired or wirelessly connected reference sensors at a location on the subject's upper torso; marking a location of at least a xiphoid process and a side of the subject utilizing a registration sensor; calculating a width of the subject's torso based on the location of the xiphoid process and the side of the torso marked by the registration sensor; determining a plane defined by signals obtained from the at least two reference sensors, and the xiphoid process marked by the registration sensor; calculate a vector N normal to the plane; and producing a diagram of the subject's upper body indicating in the location of the subject's xiphoid process and suprasternal notch, inserting an enteral tube comprising an electromagnetic sensor into the subject; and determining in real-time, by changes in the strength of the electromagnetic field sensed by the electromagnetic sensor, a location and direction of the tip of the enteral tube relative to one or both of the pair of reference sensors, and projecting its insertion path over the diagram, using vector N to determine the projection direction.

According to some embodiments, the produced diagram takes into consideration the calculated width of the subject's torso.

According to some embodiments, the method may also include marking the suprasternal notch and determining the orientation of the subject based thereon. Alternatively, the location of the suprasternal notch may be calculated, based on the location of the at least two reference sensors.

According to some embodiments, the method further includes displaying the diagram on a monitor or on another display device such as a mobile phone, a tablet etc.

According to some embodiments, the diagram shows a frontal upper view of the subject's torso, a side view of the subject's torso and/or an axial view of the subject's torso.

According to some embodiments, the location of the pair of reference sensors on the subject's upper torso is at or near the subject's claviculae.

According to some embodiments, there is provided a processing circuitry configured to: receive signals from at least two wired or wirelessly connected reference sensors positioned at a location on the subject's upper torso and exposed to an electromagnetic field; receive indications of a location of at least a xiphoid process and a side of the subject's torso (e.g. the armpit) marked by a registration sensor, the registration sensor configured to send and receive electromagnetic signals when exposed to an electromagnetic field; calculate a width of the subject's torso based on the location of the xiphoid process and the side of the subject's torso marked by the registration sensor; determine a plane defined by the at least two reference sensors, and the xiphoid process marked by the registration sensor; and calculate a vector N normal to the plane; produce a diagram of the subject's upper body indicating the location of the subject's xiphoid process and suprasternal notch.

According to some embodiments, the processing circuitry is further configured to receive signals from a enteral tube comprising an electromagnetic tip sensor, and determining in real-time, based on changes in the strength of the electromagnetic field sensed by the electromagnetic sensor, a location, direction and/or path of the tip of the enteral tube relative to one or both of the pair of reference sensors, and projecting its insertion path over the diagram, using vector N to determine the projection direction.

According to some embodiments, the processing circuitry is further configured to indicate/display the location and/or direction of the tip of the enteral tube relative to the diagram. According to some embodiments, the processing circuitry is further configured to generate an insertion path. According to some embodiments, the processing circuitry is further configured to generate determine and/or show the direction of the tip of the enteral tube. Thereby enabling the anticipating of direction to which it is headed.

According to some embodiments, the processing circuitry is further to receive an indication of a location of the suprasternal notch marked by the registration sensor. Alternatively, the processing circuitry may be configured to calculate the location of the suprasternal notch, based on the signals obtained from the at least two reference sensors.

According to some embodiments, the diagram shows a frontal upper view of the subject's torso, a side view of the subject's torso and/or an axial view of the subject's torso.

According to some embodiments, the location on the subject's upper torso is at or near the subject's claviculae.

Certain embodiments of the present disclosure may include some, all, or none of the above advantages. One or more technical advantages may be readily apparent to those skilled in the art from the figures, descriptions and claims included herein. Moreover, while specific advantages have been enumerated above, various embodiments may include all, some or none of the enumerated advantages.

### BRIEF DESCRIPTION OF THE FIGURES

Some embodiments of the disclosure are described herein with reference to the accompanying figures. The description, together with the figures, makes apparent to a person having ordinary skill in the art how some embodiments of the disclosure may be practiced. The figures are for the purpose of illustrative discussion and no attempt is made to show details of an embodiment in more detail than is necessary for a fundamental understanding of the teachings of the disclosure.
**FIG. 1** is a block diagram of an insertion device positioning guidance system, according to some embodiments;
**FIG. 2** is a flowchart of a method for guiding insertion of an insertion tube in a patient, according to some embodiments;
**FIG. 3A****-****FIG. 3D** illustrate the sensor setup and registration process, according to some embodiments.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

According to some embodiments, there is provided an enteral tube positioning guidance system comprising: an electromagnetic field generator configured to generate an electromagnetic field covering a treatment area; wherein said electromagnetic field generator is external to the patient; at least two wired or wirelessly connected reference sensors configured for positioning at a location on the subject's upper torso, wherein the at least two reference sensors are configured to sense the electromagnetic field generated by the field generator; a registration sensor configured to sense the electromagnetic field and to transmit information indicative of its location, wherein the registration sensor is utilized to mark a location of at least a xiphoid process and a side of the subject's torso; and a processing circuitry.

According to some embodiments, the processing circuitry configured to calculate a width of the subject's torso based on the location of the xiphoid process and the side of the subject's torso marked by the registration sensor; determine a plane defined by the at least two reference sensors, and the xiphoid process marked by the registration sensor; calculate a vector N normal to the plane; displaying a diagram of the subject's upper body indicating/determining the location of the subject's xiphoid process and the suprasternal notch, wherein the diagram takes into consideration the calculated width of the subject's torso.

According to some embodiments, indicating/determining the location of the subject's suprasternal notch is based on the location of the suprasternal notch as marked by the registration sensor. Additionally or alternatively, the location of the suprasternal notch may be determined/calculated based on signals obtained from the reference sensors.

According to some embodiments, the location, direction and/or path of the enteral tube comprising an electromagnetic sensor during insertion vis-a-vis the subject's suprasternal notch and xiphoid process, can be determined, based on the vector N calculated and changes in the strength of the electromagnetic field sensed by the electromagnetic sensor of the enteral tube.

The electromagnetic field generator may be static throughout a duration of a procedure for placing a tube within a body of a subject. In such cases, a region covered by the electromagnetic field is static/constant throughout the duration of a procedure for placing a tube within a body of a subject. Advantageously, the static electromagnetic field may contribute to the accuracy of the display.

One example of hardware suitable for use as the abovementioned electromagnetic tracking system, including the electromagnetic field generator and one or more of the sensors, is the Aurora^{®} system by Northern Digital Inc., of Ontario, Canada.

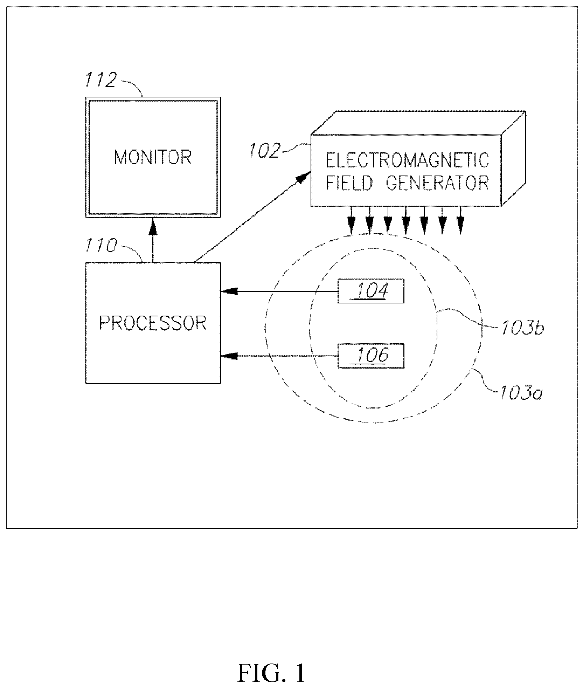

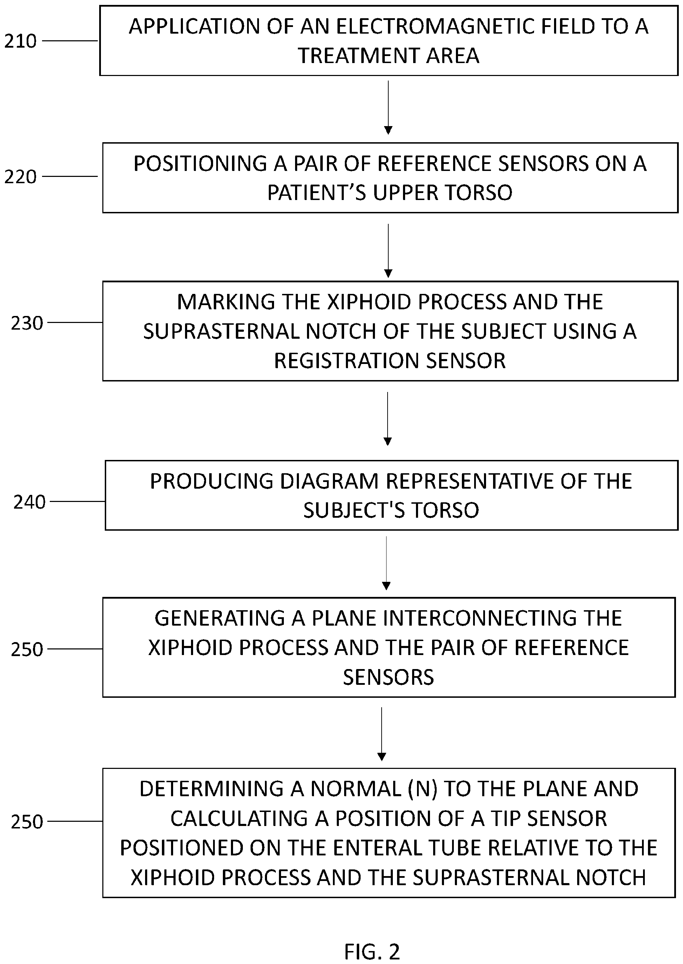

Throughout the following description, similar elements of different embodiments of the device are referenced by element numbers differing by integer multiples of 100. For example, an electromagnetic field generator of FIG. 1 is referenced by the number 102, and an electromagnetic field generator of **FIG. 2****,** which corresponds to electromagnetic field generator **102** of **FIG. 1****,** is referenced by the number **202.**

Reference is now made to **FIG. 1** which is a block diagram of an insertion device positioning guidance system **100.** System **100** includes an electromagnetic field generator **102** configured to generate an electromagnetic field **103a** covering at least a region of interest **103b** (e.g., a treatment area such as a patient's torso), a plurality of electromagnetic sensors, such as sensors **104** and **106,** on. System **100** further includes a processor **110** configured to operate said field generator, read signals obtained from reference sensors **104** and **106,** and to generate an anatomical map representing the torso of the subject. Processor **110** is configured to facilitate visualization on the anatomical map of a location, direction and/or path of the tip sensor, on the map, independent of the subject's movement and independent of deviations in the position and/or orientation of field generator **102.** System **100** further includes a monitor **112** operatively connected to processor **110** and configured to display, on the anatomical map, the location of, the direction of and/or the path of the insertion device tip, comprising an electromagnetic sensor, during its insertion. In some embodiments, monitor **112** may be integrated with processor **110,** such as in the case of an all-in-one computer or a smartphone. A determination of a successful medical procedure (for example, an insertion of a feeding tube to the stomach as opposed to the lungs) is thus possible.

Sensors **104** and **106** are configured to define a reference coordinate system representing the position and orientation of the subject's torso relative to field generator **102.** Optionally, sensors **104** and **106** may be 6-DOF electromagnetic sensors, capable of determining 6 axes of its location (XYZ axes) and angles/orientation (roll, yaw, and pitch) with respect to the electromagnetic field **103a** generated by field generator **102.**

Guidance system also includes a registration sensor (not shown) configured to mark at least one anatomic (thoracic) location over the subject's body, such as the side of the subject's torso, the xiphoid process and optionally also the suprasternal notch. Different anatomical locations may be marked depending on the type of procedure used, the type of insertion medical device, etc. The marking of the anatomic location may be physical, such as attaching a marker/fiducial (such as a sticker). Alternatively, the marking of the anatomic location may be virtual, such as registering a virtual marker/fiducial. The marking, in accordance with embodiments, may facilitate identification or designation of an anatomical location within or on a subject's body such as, in a non-limiting example, a subject's suprasternal notch and/or a subject's xiphoid process.

Optionally, registration sensor **107** is a stylus sensor having a 3 DOF sensor on its distal tip, the stylus may be configured to be manually operated to mark the anatomic location on the subject's torso as identified by the operator of the stylus. The marking may be made, merely as an example, by indicating to the software (for example, but not limited to) by pressing a GUI button or voice activation) once stylus sensor 106 is positioned over/at the desired point on the patient's torso. The marking may be communicated to and registered by processor **110.**

System **100** is configured to work in conjunction with an insertion medical device (not shown), such as a feeding tube. The insertion medical device may include one or more sensors to allow its tracking within region of interest **103b.** Preferably, the sensor is located at the tip of the insertion medical device. In such case, processor **110** and monitor **112** are configured to compute and display location, direction and/or advancement/path of the tip of the insertion medical device between the designated anatomical locations leading to the insertion site/target area.

According to some embodiments, the system may include a non-transitory computer-readable storage medium, or in other words, a memory module. According to some embodiments the memory module may have stored thereon one or more program codes configured to operate the processor and/or any one or more of the field generator **102,** any one or more of the reference sensors **104** and **106,** monitor **112,** and/or other elements of the system **100.**

According to some embodiments, the program code, or the one or more algorithms, may be executable by the processor to receive signals from any one or more of the reference sensors **104** and **106.** According to some embodiments, the program code, or the one or more algorithms, may be executable by the processor to receive signals from the registration sensor. According to some embodiments, the one or more algorithms may include one or more preprocessing methods. According to some embodiments, the one or more algorithms may be configured to apply one or more preprocessing methods to one or more signals received from any one or more of the reference sensors **104** and **106.** According to some embodiments, the one or more algorithms may be configured to apply one or more preprocessing methods to one or more signals received from the registration sensor. According to some embodiments, the one or more preprocessing methods may include any one or more of normalizing the signals, noise reduction, and the like.

According to some embodiments, the one or more algorithms may include one or more machine learning algorithms configured to identify the xiphoid process. According to some embodiments, the one or more algorithms may include one or more machine learning algorithms configured to identify the suprasternal notch. According to some embodiments, the machine learning algorithm may be configured to identify a predetermined location of the patient's upper torso, in real time.

According to some embodiments, the machine learning algorithm may include a supervised machine learning algorithm. According to some embodiments, the machine learning algorithm may include an unsupervised machine learning algorithm. According to some embodiments, the training set for training of the machine learning algorithm may include a data set of images associated with patients' upper torsos, such as, for example, X-ray, MRI, and/or ultrasound images. According to some embodiments, the training set for training of the machine learning algorithm may include a data set of signals received from registration sensors and/or reference sensors. According to some embodiments, the training set may include labels indicating locations of specified locations of the patient's upper torso, such as, for example, the xiphoid process and/or the suprasternal notch.

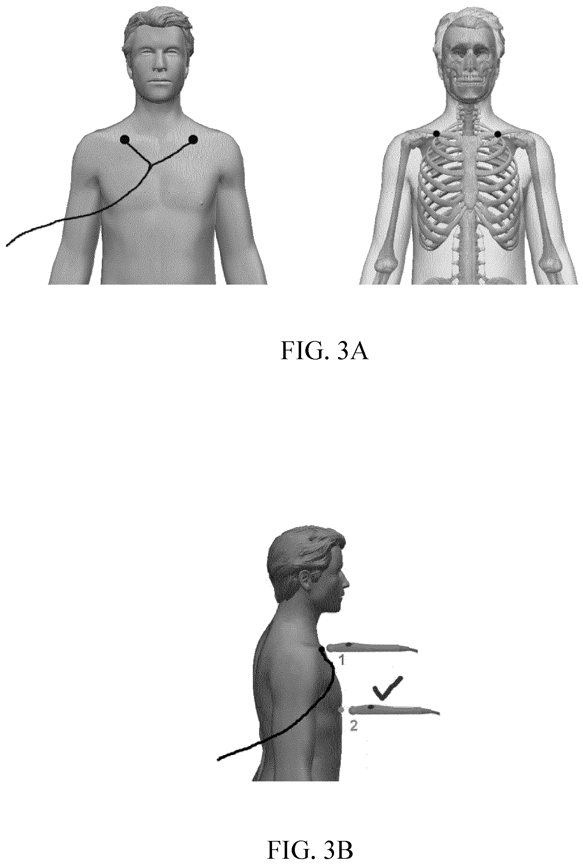

Reference is now made to **FIG. 2** and to **FIG. 3A****-****FIG.3E****,** which depict the steps of the guiding of insertion of an enteral tube, in accordance with some embodiments. It is understood to one of ordinary skill in the art that while some steps are sequential others may be performed simultaneously or in an interchangeable order and these options are within the scope of this disclosure.

Step **210** includes application of an electromagnetic field to a treatment area. Optionally, an electromagnetic field generator is positioned such that the electromagnetic field covers the treatment area. A pair of reference sensors is positioned on a patient's upper torso, e.g. at or near each of the patient's claviculae, as shown in **FIG. 3A****.** According to some embodiments, the reference sensors define a reference coordinate system representing the position and/or orientation of the subject's torso relative to the field generator (step **220**). In step **230** a registration sensor (e.g. in the form of a manually operated stylus) is utilized to mark of the xiphoid process and optionally also the suprasternal notch, as shown in **FIG. 3B****.** Alternatively, the location of the suprasternal notch may be calculated based on the pair of reference sensors positioned at or near the subject's claviculae. (option not shown).

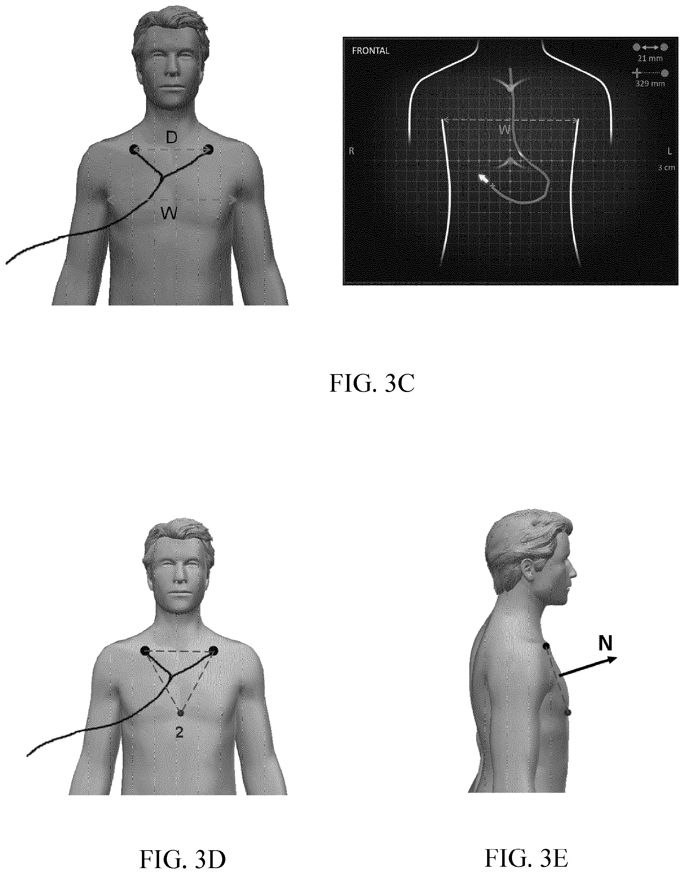

A diagram representative of the subject's torso may then be produced based on the signals obtained from the pair of reference sensors and on the width of the subject, determined based on the marking of the side(s) of the subject's torso by the registration sensor (step **240**), as shown in **FIG. 3C****.**

A plane (tringle) may then be generated/displayed, interconnecting the xiphoid process and the two reference sensors (step **250**), as shown in **FIG. 3D****.**

A normal **N** to the plane may then be calculated (N pointing in the same general direction as the registration sensor during setup), as shown in **FIG. 3E****.** The normal N is then used to calculate the location, direction and/or path of a tip sensor (positioned on the tip of an enteral tube) (step **260**), assuming a fixed angle between the xiphoid process and the suprasternal notch, namely approximately 15 degrees.

Advantageously, movement of one or more of the pair of reference sensors can be easily detected by a rapid change in the distance between the sensors.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire. Rather, the computer readable storage medium is a non-transient (i.e., not-volatile) medium.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. An enteral tube positioning guidance system comprising:
an electromagnetic field generator configured to generate an electromagnetic field covering a treatment area; wherein said electromagnetic field generator is external to the patient;
at least two wired or wirelessly connected reference sensors configured for positioning at a location on the subject's upper torso, wherein the at least two reference sensors are configured to sense the electromagnetic field generated by the field generator;
a registration sensor configured to sense the electromagnetic field and to transmit information indicative of its location, wherein the registration sensor is utilized to mark a location of at least a xiphoid process and a point on side of the subject's torso; and
a processing circuitry configured to:
calculate a width of the subject's torso based on the location of the xiphoid process and the point on the side of the subject's torso marked by the registration sensor;
determine a plane defined by the at least two reference sensors, and the xiphoid process marked by the registration sensor;
calculate a vector N normal to the plane; and
displaying a diagram of the subject's upper body indicating the location of the subject's xiphoid process and suprasternal notch,
wherein the diagram takes into consideration the calculated width of the subject's torso and the location of one or both of the pair of reference sensors, and
determining in real-time, by changes in the strength of the electromagnetic field sensed by the electromagnetic sensor, a location, direction and/or insertion path of a tip of the enteral tube relative to one or both of the pair of reference sensors, and projecting the location, direction and/or insertion path over the diagram, using vector N to determine the projection direction.

2. The system of claim 1, wherein the registration sensor is further configured to mark the suprasternal notch.

3. The system of claim 1, wherein the diagram shows a frontal upper view of the subject's torso, a side view of the subject's torso and/or an axial view of the subject's torso and the location, direction and/or insertion path of the tip of the enteral tube in each view, as calculated by tracking the location of the tip sensor.

4. The system of claim 1, further comprising a monitor configured to display the diagram.

5. The system of claims 1, wherein the registration sensor is incorporated into a tip of a stylus configured to be manually operated.

6. The system of claim 1, wherein the processing circuitry is further configured to calculate the position of the suprasternal notch based on the signals obtained from the at least two reference sensors.

7. The system of claim 1, wherein the location on the subject's upper torso is at or near the subject's claviculae.

8. A method for guiding insertion of an enteral tube into a subject, the method comprising:
applying an electromagnetic field covering at least part of a subject's torso utilizing an external electromagnetic field generator;
positioning at least two wired or wirelessly connected reference sensors at a location on the subject's upper torso;
marking a location of at least a xiphoid process and a point on a side if the subject's torso utilizing a registration sensor;
calculating a width of the subject's torso based on the location of the xiphoid process and the point on the side of the subject's torso marked by the registration sensor;
determining a plane defined by signals obtained from the at least two reference sensors, and the xiphoid process marked by the registration sensor;
calculate a vector N normal to the plane;
producing a diagram of the subject's upper body indicating the location of the subject's xiphoid process and suprasternal notch, wherein the diagram takes into consideration the calculated width of the subject's torso and the location of one or both of the pair of reference sensors;
inserting an enteral tube comprising an electromagnetic sensor into the subject; and
determining in real-time, by changes in the strength of the electromagnetic field sensed by the electromagnetic sensor, a location, direction and/or insertion path of a tip of the enteral tube relative to one or both of the pair of reference sensors, and projecting the location, direction and/or insertion path over the diagram, using vector N to determine the projection direction.

9. The method of claim 8, further comprising marking the suprasternal notch.

10. The method of claim 8, wherein the diagram shows a frontal upper view of the subject's torso, a side view of the subject's torso and/or an axial view of the subject's torso.

11. The method of claim 8, further comprising calculating the position of the suprasternal notch based on the signals obtained from the at least two reference sensors.

12. The method of claim 1, wherein the location on the subject's upper torso is at or near the subject's claviculae.

13. The method of claim 1, further comprising displaying in real-time the position of the enteral tube on the diagram.

14. A processing circuitry configured to:
receive signals from at least two wired or wirelessly connected reference sensors positioned at a location on the subject's upper torso and exposed to an electromagnetic field;
receive indications of a location of at least a xiphoid process and a point on a side of the subject's torso marked by a registration sensor, the registration sensor configured to sense an electromagnetic field generated by a field generator;
calculate a width of the subject's torso based on the location of the xiphoid process and the point on the side of the subject's torso marked by the registration sensor;
determine a plane defined by the at least two reference sensors, and the xiphoid process marked by the registration sensor;
calculate a vector N normal to the plane;
produce a diagram of the subject's upper body indicating in the location of the subject's xiphoid process and suprasternal notch,
receive signals from an enteral tube comprising an electromagnetic tip sensor,
determine in real-time, by changes in the strength of the electromagnetic field sensed by the electromagnetic sensor, a location, direction and/or insertion path of a tip of the enteral tube relative to one or both of the pair of reference sensors, and projecting the location, direction and/or insertion path over the diagram, using vector N to determine the projection direction.

15. The processing circuitry of claim 14, wherein the processing circuitry is further configured to receive an indication of a location of the suprasternal notch marked by the registration sensor.

16. The processing circuitry of claim 14, wherein the diagram shows a frontal upper view of the subject's torso, a side view of the subject's torso and/or an axial view of the subject's torso.

17. The processing circuitry of claim 14, further configured to calculate the position of the suprasternal notch, based on the signals obtained from the at least two reference sensors.

18. The processing circuitry of claim 14, wherein the location on the subject's upper torso is at or near the subject's claviculae.
